# EUROPEAN PATENT APPLICATION

(11) **EP 1 946 750 A1**
(43) Date of publication of application: **23.07.2008**
(21) Application number: 07000977.4
(22) Date of filing: 18.01.2007
(51) Int. Cl.: A61K 9/20, A23L 1/164

(54) **Co-precipitate, method for preparing the same and uses thereof**

(71) Applicant: The Jordanian Pharmaceutical Manufacturing Co., 11710 Naor (JO)
(72) Inventor: Badwan, Adnan, 11185 Amman (JO); Al-Remawi, Mayyas, 13713 Russiefa (JO); Rashid, Daid Iyad, 11151 Amman (JO)
(74) Representative: Winkler, Andreas Fritz Ernst

(57) **Abstract**

The invention relates to a co-precipitate comprising at least one hydrophilic polymeric compound selected from the group consisting of xanthan gum, sodium alginate, propylene glycol alginate, pectin, guar gum, cellulose, sodium carboxymethyl cellulose, hydroxymethyl propyl cellulose, methyl cellulose, hydroxypropyl cellulose, hydroxyethyl cellulose, cyclodextrins, tragacanth, locust bean gum, carrageenan, polydextrose, dextrin, maltodextrin, polyethylene glycol, polyethylene oxide, polyvinyl alcohol, povidone, microcrystalline cellulose, croscarmellose sodium, calcium carboxymethyl cellulose, crospovidone, alginic acid and docosate sodium, or any other salts, solvates, derivatives or mixtures thereof, and at least one water-absorbable compound selected from the group consisting silicon dioxide, derivatives thereof, or any mixtures thereof; a method of preparing the same and uses thereof.

## Description

### Field of Invention

The present invention relates to a co-precipitate, a method for preparing the same as well as an use of the co-precipitate as a filler and/or a disintegrant for solid pharmaceutical dosage forms.

### Background of the Invention

Solid pharmaceutical dosage forms consist in most cases of powder mixtures of active material and pharmaceutical excipients.

The excipients can be classified into the following:

Excipients that are necessary for maintaining the physical structure of the solid dosage form before the patient intake including diluents, binders, and lubricants.

Excipients which help to give additional desirable physical characteristics to the finished tablet including colors, and, in the case of chewable tablets, flavors, sweetening agents, and coating materials.

Excipients playing a role during tablet formation are called filler/binder or excipient needed for bond formation. It is a substance added to facilitate formation of a hard tablet upon compaction of the powder formula especially with poorly compactable active materials. Fillers usually have powerful flow and compaction properties such as microcrystalline cellulose.

Excipients that play a role after the ingestion of the solid dosage form are called disintegrants. It is a substance, or a mixture of substances, added to a tablet to facilitate its breakup or disintegration after administration. The active ingredient must be released from the tablet matrix as efficiently as possible to allow for a rapid dissolution. Materials serving as disintegrants have been chemically classified as starches, clays, celluloses, algins, or gums.

The most popular disintegrants are corn and potato starch which have been well-dried and powdered.

Starch consists of two polysaccharides, amylose and amylopectin. Starch is well known to be used as a glidant; tablet binder; tablet and capsule diluent; tablet and capsule disintegrant.

Starch suffers from bad flowability, incompressibility, and poor disintegration power. However, starch is considered as a cheap excipient.

Due to its low price, many attempts have been proposed to solve these problems. For example, to solve the problem of poor disintegration power, starch derivatives were prepared. One of the most important and widely used is the sodium starch glycolate also known as carboxymethyl starch or primojel.

Sodium starch glycolate is widely used in oral pharmaceuticals as a disintegrant in capsule and tablet formulations. The usual concentration employed in a formulation is between 2-8% w/w, with the optimum concentration about 4% although in many cases 2% is sufficient. Disintegration occurs by rapid uptake of water followed by rapid and enormous swelling. However, this starch derivative suffers from bad flowability and relatively poor compactability.

Another starch derivative is pregelatinized starch. It is a starch that has been chemically and/or mechanically processed to rupture all or part of the starch granules and so render the starch flowable and directly compressible. However, this starch suffers from relatively lower disintegration power than sodium starch glycolate. The concentration to be used as a tablet disintegrant is in the range of 5-20% w/w.

Other non-starch superdisintegrants and their used percentages to achieve disintegration of tablets include: powered cellulose 5-15%, microcrystalline cellulose 5-15%, croscarmellose sodium 0.5-5%, calcium carboxymethyl cellulose 1-15%, crospovidone 2-5%, alginic acid 1-5%, high viscosity grades of methyl cellulose 2-10%, low-substituted hydroxypropyl cellulose 5-25%, and docusate sodium 0.5%.

To achieve superdisintegration the previously known superdisintegrants should be generally used in low percentages in the solid pharmaceutical dosage forms. Many of these superdisintegrants also suffer from poor flowability and/or compactability.

It is an object of the present invention to provide excipients for solid pharmaceutical dosage forms which overcome the drawbacks of the prior art, which show particularly improved disintegration power, flowability and compactability. Further, a method for preparing such excipients and uses thereof shall be provided.

The first object is achieved by a co-precipitate according to claim 1. A method for its preparation is disclosed in claim 7, whereas use of such a co-precipitate is disclosed in claim 10. Preferred embodiments are disclosed in the sub-claims.

Surprisingly, the novel co-precipitate improved both the disintegration power and the powder physical properties including flowability and compactability more than the previously known excipients. The inventive method enables the use of a single tabletting material that serves two purposes in one substance, i.e. superdisintegrant and filler at the same time.

According to the present invention, hydrophilic polymers are understood to comprise polymers that hydrate in an aqueous medium and form a gel structure. These polymers may be cationic, anionic and non-ionic in chemical nature. Preferably, silica-based substances can be used as a water absorbing compound. The co-precipitation of especially silica-based substances with the hydrophilic polymers was found to improve the physical and disintegration properties of the produced mixture.

The co-precipitate also has an increased power for water absorption compared to known excipients. Another surprising effect is the powerful improvement in flow and compaction of the inventive co-precipitate excipients compared to each component when taken alone.

Particularly suitable hydrophilic polymers for use herein include water-soluble or water-insoluble, covalently bonded derivatives or ionically bonded derivatives known in the art.

Silicon dioxide exists in a variety of crystalline forms and amorphous modifications, any of which are suitable for use herein. In particular silicon dioxide having a high surface area according to BET is preferred, such as colloidal silica.

The inventive co-precipitate can be used as filler and/or disintegrant, preferably in immediate release solid pharmaceutical dosage forms.

The inventive co-precipitate, when compressed, using any compression machine known in the art, forms a strong compact. This compact exhibits a superdisintegration power when exposed to aqueous medium.

Additional features and advantages of the subject-matter of the present invention will become apparent from the following detailed description of examples.

### EXAMPLES

### Example 1

### Use of silicated pectin as a superdisintegrant and/or filler

Pectin was physically mixed with aerosil in a ratio of 1:1, then granulated with deionised water prior to drying and sieving.

Silicated pectin was added at different percentages of 1, 5, 10 and 20% to microcrystalline cellulose (Avicel^{®} 200) to formulate a total tablet weight of 500 mg. The tablets were compressed using a single press tableting machine at a pressure of 1 ton and using a circular punch of 12 mm diameter. Tablet hardness was around 300 kN.

**Table 1: Disintegration time of different silicated pectin percentages in a tablet containing microcrystalline cellulose as a filler (Avicel 200)**

| | Disintegration time at different percentages | | | | |
|---|---|---|---|---|---|
| Material | 1% | 5% | 10% | 20% | 100% |
| Avicel 200(Blank) | > 10 minutes | | | | |
| Pectin-silica | <50 sec | <45 sec | <30 sec | <10 sec | <5 sec |

### Example 2

### Use of silicated gum arabicum as a superdisintegrant and/or filler

Equal masses of gum arabicum (commercial grade) and colloidal silica (1:1 w/w) were physically mixed and granulated with deionized water and then dried in an oven at 80°C. The formula was then sieved over a 425 µm mesh. The disintegration power of gum-silica product was investigated by incorporating the product with microcrystalline cellulose (Avicel^{®} 200) at various percentages and pressing them using 12 mm planar punch of 500 mg weight. The compression force was fixed at about 45 kN using a single punch tableting machine. Tablet hardness was greater than 90 N.

**Table 2: Disintegration time of different silicated gum arabicum percentages in a tablet containing microcrystalline cellulose as a filler (Avicel® 200)**

| | | | | | |
|---|---|---|---|---|---|
| Gum Arabicum content | 1% | 5% | 10% | 20% | 100% |
| Disintegration time, sec | <40 | <25 | <20 | <10 | <3 |

### Example 3

### Use of silicated polyethylene glycol (PEG) as a superdisintegrant and/or filler

PEG was physically mixed with colloidal silicon dioxide (Aerosil ®200) in the ratio of 1:1.67, the mixture was then granulated with deionised water. Silicated PEG was added at different percentages of 1, 5, 10, and 20% to Avicel® 200 to formulate a total tablet weight of 500 mg. The tablets were compressed using the single press tableting machine at a pressure of 1 ton and using a circular punch of 12 mm diameter. Tablet hardness was greater than 90 kN.

**Table 3: Disintegration time of different silicated polyethylene glycol percentages in a tablet containing microcrystalline cellulose as a filler (Avicel® 200)**

| | Disintegration time at different percentages | | | | |
|---|---|---|---|---|---|
| Material | 1% | 5% | 10% | 20% | 100% |
| Avicel® 200(Blank) | >10 minutes | | | | |
| PEG-silica | <1.5 min | <1.2 min | <50 sec | <40 sec | <10 sec |

### Example 4

### Use of silicated powder cereal products as a compactable rapidly disintegrable infant nutrient

Cereal powders (contains hydrophilic polymers) usually become physically sticky as soon as they absorb water from the surrounding medium, giving no apparent disintegration.

Granulation was the most desired process for the distribution of disintegrants within the granules. The granulated formulae were dried at 45 °C, then compressed using a Manesty single punch tabletting machine using a 12 mm circular punch. The resulting tablets were used to measure the disintegration time in water. The compression force was varied from 30-50 kN. Table 4 below summarises the materials used with results of disintegration testing:

**Table 4: The effect of a number of disintegrants on the disintegration of cereals.**

| Disintegrating material | % of disintegrant within the cereal | Granulating medium | Disintegration time | Hardness, N |
|---|---|---|---|---|
| Cereal-Silica | 10-15 | Water | No disintegration | 50-100 |
| Cereal-Silica | 10-15 | Ethanol | <1 minute | 50 |
| | | | 1-2 minutes | 70 |

The features disclosed in the foregoing description and in the claims may, both separately and in any combination thereof, be material for realising the invention in diverse forms thereof.

## Claims

1. Co-precipitate comprising at least one hydrophilic polymeric compound selected from the group consisting of xanthan gum, sodium alginate, propylene glycol alginate, pectin, guar gum, cellulose, sodium carboxymethyl cellulose, hydroxymethyl propyl cellulose, methyl cellulose, hydroxypropyl cellulose, hydroxyethyl cellulose, cyclodextrins, tragacanth, locust bean gum, carrageenan, polydextrose, dextrin, maltodextrin, polyethylene glycol, polyethylene oxide, polyvinyl alcohol, povidone, microcrystalline cellulose, croscarmellose sodium, calcium carboxymethyl cellulose, crospovidone, alginic acid, docusate sodium and cereal powder, or any other salts, solvates, derivatives or mixtures thereof, and at least one water-absorbable compound selected from the group consisting silicon dioxide, derivatives thereof, or any mixtures thereof.

2. Co-precipitate according to claim 1, wherein the water-absorbable compound is selected from the group consisting of silicon dioxides and silicates.

3. Co-precipitate according to claim 2, wherein the water-absorbable compound is selected from the group consisting of colloidal silicon dioxide, magnesium aluminum silicate, magnesium trisilicate, bentonite and kaolin.

4. Co-precipitate according to any of the preceding claims, wherein the amount of the water-absorbable compound in the co-precipitate is in the range of 1-90% w/w, pref erably 40-60% w/w.

5. Co-precipitate according to any of the preceding claims, wherein the water-absorbable compound has a surface area according to BET from about 5 to about 300 m²/g, preferably 10-250 m²/g, most preferably 15 to 200 m²/g.

6. Co-precipitate according to any of the preceding claims, further comprising additional agents selected from the group consisting of fillers, lubricants, pH-buffering agents and coloring agents.

7. Method for preparing a co-precipitate according to any of the preceding claims by mixing a wet mass of the hydrophilic polymeric compound with a suspension or solution of the water-absorbable compound in an appropriate ratio and adjusting the process conditions in order to allow the co-precipitate to form.

8. Method according to claim 7, wherein the pH of the mixing solution is between 4 and 10, preferably between 4 and 7.

9. Method according to claim 7 or 8, wherein the method for co-precipitation is accomplished by wet or dry granulation, pH change co-precipitation, spray drying freeze drying or simple solution mixing.

10. Use of a co-precipitate according to any of the claims 1 to 7 as a filler and/or disintegrant for solid pharmaceutical dosage forms.
